# EUROPEAN PATENT APPLICATION

(11) **EP 4 215 558 A1**
(43) Date of publication of application: **26.07.2023**
(21) Application number: 21868677.2
(22) Date of filing: 16.09.2021
(51) Int. Cl.: C08G 18/66, C08G 18/48, C08G 18/32, C08G 18/12, A61F 6/04

(54) **AQUEOUS POLYURETHANE EMULSION, PREPARATION METHOD THEREFOR AND USE THEREOF**

(30) Priority: 21.09.2020 CN 202010997942
(71) Applicant: Reckitt Benckiser Health Limited, Slough, Berkshire SL1 3UH (GB)
(72) Inventor: DAI, Jiabing, Lanzhou, Gansu 730000 (CN); FENG, Linlin, Lanzhou, Gansu 730000 (CN); LIU, Bin, Lanzhou, Gansu 730000 (CN); CHEN, Liang, Lanzhou, Gansu 730000 (CN)
(74) Representative: O'Brien, Niall James
(86) International application number: PCT/CN2021/118732
(87) International publication number: WO 2022/057855

(57) **Abstract**

The invention provides a waterborne polyurethane emulsion and preparation method and use thereof. The method comprises the steps of: heating and dehydrating a macromolecular diol, and then reacting with a first polyisocyanate to obtain a first prepolymer; reacting the first prepolymer with a second polyisocyanate to obtain a second prepolymer; reacting the second prepolymer with a hydrophilic chain extender and a small-molecule chain extender to obtain a third prepolymer; reacting the third prepolymer with a salt forming agent, followed by emulsification upon addition of a post-chain extender, and by desolvation to obtain a polyurethane emulsion; wherein the molar amount of the first polyisocyanate is less than that of the second polyisocyanate, and the molar ratio of the first polyisocyanate to the macromolecular polyol is less than 1:3. The condom prepared using the polyurethane emulsion of the present invention has the characteristics of high elasticity and high strength.

## Description

### Technical field

The present invention relates to the technical field of waterborne polyurethane application, and in particular to a waterborne polyurethane emulsion and a preparation method and use thereof.

### Background

Condom is currently the most widely used and simple device for contraception and prevention of sexually transmitted diseases in the world. Existing products mainly include natural latex rubber condoms and waterborne polyurethane condoms. At present, the polyurethane condoms on the market generally have low strength and poor resilience. Since the traditional waterborne polyurethane emulsion is prepared by a three-step or one-step method, the condoms made by these methods have disadvantages such as poor resilience and low strength. Therefore, it is of great commercial significance to develop a waterborne polyurethane condom with high resilience and high strength starting from the step of polyurethane synthesis, so as to further improve the performance advantages of the polyurethane condom products over traditional latex condoms.

### Summary of the Invention

In order to solve the technical problems existing in the prior art, the present invention provides a waterborne polyurethane emulsion and a preparation method and use thereof. The polyurethane condom prepared by the polyurethane emulsion according to the present invention has high resilience and high strength performances.

First, the present invention provides a method for preparing a waterborne polyurethane emulsion, comprising at least the steps of: heating and dehydrating a macromolecular polyol, and then reacting with a first polyisocyanate to obtain a first prepolymer; reacting the first prepolymer with a second polyisocyanate to obtain a second prepolymer; reacting the second prepolymer with a hydrophilic chain extender and a small-molecule chain extender to obtain a third prepolymer; and reacting the third prepolymer with a salt forming agent, followed by emulsification upon addition of a post-chain extender, and by desolvation, to obtain a polyurethane emulsion; wherein the molar amount of the first polyisocyanate is less than that of the second polyisocyanate, and the molar ratio of the first polyisocyanate to the macromolecular polyol is less than 1:3.

In one embodiment, the macromolecular polyol comprises a macromolecular diol and a macromolecular triol, and the mass ratio of the macromolecular diol to the macromolecular triol is (10-15):(1.5-3.5).

In one embodiment, the molecular weight of the macromolecular diol is 1,000 to 3,000, and the molecular weight of the macromolecular triol is 600 to 6,000.

In one embodiment, the molar ratio of the macromolecular polyol, the first polyisocyanate and the second polyisocyanate is (1-1.5):(0.2-0.3):(2-2.5).

In one embodiment, the molar ratio of the hydrophilic chain extender, the small-molecule chain extender and the post-chain extender is (1-1.3):(0.01-0.05):(0.5-0.9).

In one embodiment, the R value in the waterborne polyurethane emulsion is 1.4-1.6, and the R value is the molar ratio of cyanate groups to hydroxyl groups in the waterborne polyurethane emulsion.

Another aspect of the present invention further provides a waterborne polyurethane emulsion prepared by the method described above.

In one embodiment, the waterborne polyurethane emulsion is an anionic waterborne polyurethane emulsion, and the solid content of the waterborne polyurethane emulsion is 30%-35%.

A further aspect of the present invention provides a waterborne polyurethane condom prepared from the polyurethane emulsion described above.

In one embodiment, the waterborne polyurethane condom has any one or more of the following characteristics:
a thickness of 0.015 to 0.025 mm;
a burst pressure of greater than or equal to 3.0 kPa; and
a tensile strength of greater than or equal to 45 N/mm².

As mentioned above, the present invention discloses a polyurethane emulsion for high-resilience and high-strength condoms synthesized by a four-step method, and a preparation method thereof. The present application prepares a waterborne polyurethane resin with high strength and high resilience, by dividing the first step in the traditional polyurethane resin synthesis process into two steps, and feeding polyisocyanate in two steps so that the soft segments in the macromolecular segments have more skeletons.

### Brief Description of Drawings

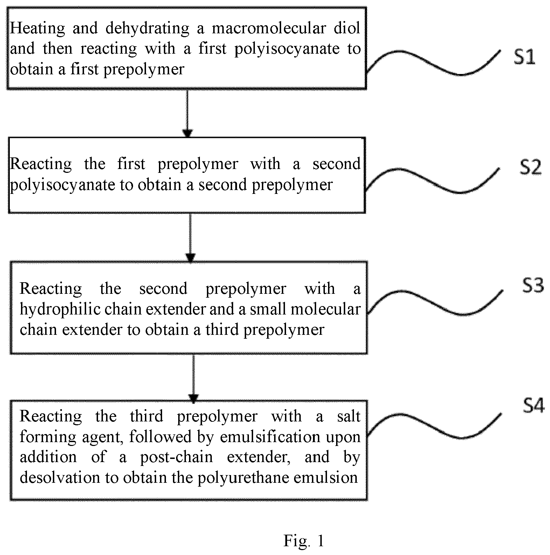

Fig. 1 shows a schematic flow chart of the preparation method of the present invention.

### Detailed Description of the Invention

Embodiments of the present invention are described below through specific examples, and those skilled in the art can easily understand other advantages and effects of the present invention from the disclosure in the specification. The present invention can also be carried out or applied through other distinct specific embodiments, and various modifications or changes can be made to the details in the specification based on different viewpoints and applications without departing from the spirit of the present invention. Note that the performance tests of the polyurethane resin and the condom of the present invention are carried out with reference to national standards GB/T1040.1-2006 and GB7544-2009, respectively. "%" and "part(s)" shown herein mean "% by mass" and "part(s) by mass", respectively, unless specified otherwise.

Reference is made to Fig. 1. First, the present invention provides a method for preparing a waterborne polyurethane emulsion, which may be carried out by repetitively dipping into a waterborne polyurethane resin, and comprises at least steps S1-S4:
S1: heating and dehydrating a macromolecular polyol, and then reacting with a first polyisocyanate to obtain a first prepolymer;
S2: reacting the first prepolymer with a second polyisocyanate to obtain a second prepolymer;
S3: reacting the second prepolymer with a hydrophilic chain extender and a small-molecule chain extender to obtain a third prepolymer; and
S4: reacting the third prepolymer with a salt forming agent, followed by emulsification upon addition of a post-chain extender, and by desolvation to obtain a polyurethane emulsion;
wherein the molar amount of the first polyisocyanate is less than that of the second polyisocyanate, and the molar ratio of the first polyisocyanate to the macromolecular polyol is less than 1:3.

In step S1, the macromolecular polyol may include a macromolecular diol and a macromolecular triol. The heating and dehydrating may be carried out in a four-necked flask, and the heating and dehydrating may be carried out under a vacuum condition. The heating temperature may be 115-120°C, and the vacuum condition may be at -0.1 MPa. The dehydrating may be dehydrating the polyol to a moisture content of less than 0.02%. Cooling may be carried out after the heating and dehydrating, and may be conducted to lower the temperature to 40°C or below. After the cooling, the first polyisocyanate may be fed into the four-necked flask for reaction. The reaction temperature may be 80-90°C, and the reaction time may be 2-3 hours. After the reaction, cooling may be further carried out, and the cooling after the reaction may also be conducted to lower the temperature to 40°C or below.

In step S1, the first polyisocyanate may be one or more of isophorone diisocyanate, diphenylmethane diisocyanate, and hexamethylene diisocyanate. The molar ratio of the macromolecular polyol, the first polyisocyanate and the second polyisocyanate may be (1-1.5):(0.2-0.3):(2-2.5). The macromolecular diol may be any one of polyoxypropylene glycol (PPG) and poly(tetramethylene ether glycol) (PTMEG) or a combination of both, and the molecular weight of the polyoxypropylene glycol may be 1,000-3,000, such as 1,000, 2,000, or 3,000. The molecular weight of the poly(tetramethylene ether glycol) may be 1,000-3,000, such as 1,000, 2,000, or 3,000.

The macromolecular triol may be polyoxypropylene triol, and the molecular weight of the polyoxypropylene triol may be 600 to 6,000. The use of this triol can improve the resilience performance of the waterborne polyurethane and simultaneously improve the strength of the condom. In the present invention, the use of the polyol having the above-mentioned molecular weight can ensure a good performance of the prepared waterborne polyurethane emulsion. If the molecular weight is too high, the resilience and breaking strength of the film formed from the waterborne polyurethane emulsion will be decreased. If the molecular weight is too small, the film formed from the waterborne polyurethane emulsion tends to crack, and the modulus is high.

In step S2, the component of the second polyisocyanate may be the same as the component of the first polyisocyanate; the molar amount of the first polyisocyanate may be less than that of the second polyisocyanate; and the molar ratio of the first polyisocyanate to the macromolecular polyol is less than 1:3. In the present invention, when the molar amounts of the first polyisocyanate and the macromolecular polyol are controlled within the above range, the viscosity in the following reactions can be well controlled to avoid the risk of prepolymer gelling.

By adding the polyisocyanate in separate steps such that the amount of polyisocyanate added for the first time is less than the amount of polyisocyanate added for the second time, the present invention allows individual polyisocyanate to be sandwiched among soft segments, instead of being distributed at both ends of the soft segments, after the first isocyanate addition; after the second isocyanate addition, long hard segments of isocyanate-hydrophilic chain extender-small-molecule alcohol sandwiched among the soft segments in the polyurethane structure are reduced, so as to reduce the phase separation between soft segments and hard segments and have a good resilience.

In step S2, the reaction time may be 2-3 hours, and the reaction temperature may be 80-90°C. Cooling may be further carried out after the reaction, and may be conducted to lower the temperature to 40°C or below. In step S3, the hydrophilic chain extender may be one or both of dimethylol propionic acid and dimethylol butyric acid; the small-molecule polyol chain extender may be one or more selected from ethylene glycol, propylene glycol, butanediol, hexanediol, and trimethylolpropane; the reaction time may be 3-4 hours; and the reaction temperature may be 80-90°C. Cooling may be carried out after the reaction, and may be conducted to lower the temperature to 50°C or below. After the cooling, a catalyst and a solvent can be further added to carry out a catalytic reaction. The catalytic reaction may be conducted at a temperature of 70-75°C, with a duration of 2-3 hours. Cooling may also be carried out after the catalytic reaction, and may be conducted to lower the temperature to 15-20°C. The solvent may be acetone.

In steps S1 to S3, the reaction can be carried out under stirring.

In step S4, the catalyst may be selected from organotins. The salt forming agent may be triethylamine, and the water may be deionized water. The post-chain extender may be a small-molecule diamine, and the small-molecule diamine may be one or more selected from ethylenediamine, hexamethylenediamine and isophoronediamine. The emulsification may be carried out in an emulsifier, and the emulsification may be carried out under stirring. The stirring speed may be 1000-1400 r/min. During the emulsification, an ice-water mixture may be added. The mass fraction the ice-water mixture may be 50-55%. The post-chain extender may be added after the ice-water mixture. When the post-chain extender is added, the stirring speed may be 300-400 r/min, for example, 350 r/min. The reaction time for the post-chain extender may be 3-4 hours. The desolvation temperature may be 40-45°C, and the desolvation pressure may be -0.07 to -0.09 MPa.

The present invention also provides a polyurethane emulsion prepared by the method described above. The polyurethane emulsion is an anionic waterborne polyurethane emulsion, and the solid content of the polyurethane emulsion is 30 to 35%. By using the polyurethane emulsion with the above solid content, the prepared polyurethane product can have a small surface tension and a moderate viscosity. The solid content of the polyurethane emulsion according to the present invention should not be too low. Otherwise, defects such as coating film shrinkage pores will appear. The pH value of the polyurethane emulsion may be 6.5-7.5.

In one embodiment, the R value (the ratio of cyanate to hydroxyl) of the polyurethane emulsion according to the present invention can be controlled at about 1.4 to 1.6, for example 1.5, since the water resistance of a condom is relatively good at around 1.5, and the moisture absorption performance will not deteriorate significantly at a later stage. Due to the limitation by the thickness of the condom, tiny defects will be magnified. When the R value is less than 1.5, the viscosity of the system is high, and the emulsification effect is poor; furthermore, with a smaller R value, the soft segment content is higher, resulting in less physical crosslinking sites among macromolecules after film formation and enhanced water absorption. If the R value is too large, the residual NCO content in the system will increase, and the emulsion particle size will become larger after reacting with water to a certain degree of crosslinking, which affects the continuity of the film formation later on.

The present invention also provides the use of the polyurethane emulsion as described above. The polyurethane emulsion according to the present invention is an anionic waterborne polyurethane emulsion with a solid content of 30% to 35% and a pH value of 6.5 to 7.5, and can be used for the production of safe and eco-friendly condoms. The thickness of the condom can be 0.015-0.025mm, the burst pressure can be greater than or equal to 3.0 kPa, and the tensile strength of the condom can be greater than or equal to 45 N/mm².

The present invention will be described in more details by means of specific examples.

### Examples

### Example 1

7.5 kg PPG (1000) and 15 kg PTMEG (2000) and polyoxypropylene triol with a molecular weight of 3,000 were fed into a reactor, heated to 110°C under stirring, dehydrated under vacuum of -0.1 MPa to a moisture content of polyoxypropylene triol of 0.03% or less, and cooled to 40°C. 0.64 kg IPDI and 0.45 kg MDI were fed thereto, heated to 85°C under stirring, maintained at the temperature and reacted for 2 hours, and cooled to 40°C. 4.99 kg IPDI and 1.2 kg MDI were fed thereto, heated to 85°C under stirring, maintained at the temperature and reacted for 2 hours, and cooled to 40°C. 0.66 kg DMPA and 6.5 kg acetone were fed thereto, heated to 80°C under stirring and reacted for 2 hours, and cooled to 50°C. 0.04 kg of a catalyst and 4.3 kg acetone were fed thereto, heated to 75°C under stirring and reacted for 3 hours, and then cooled to 15°C. 0.52 kg triethylamine and 15 kg acetone were added thereto, and stirred for 15 min to obtain a prepolymer.

The prepolymer was transferred to an emulsifier, and the rotation speed of the rotating disk was adjusted to 1300 r/min. 82 kg of an ice-water mixture was added to the prepolymer at a constant speed under high-speed stirring. Stirring was continued for 5 minutes upon opening of the prepolymer, and then the rotation speed was adjusted to 400 r/min. 0.3 kg of a 10X aqueous solution of ethylenediamine was added, and the mixture was stirred for 3 hours and then kept still for 24 hours.

The above emulsion was heated to 40°C, and the acetone in the emulsion was removed under the condition of -0.09 MPa, to prepare a waterborne polyurethane emulsion sample 1, with an R value of 1.46.

### Example 2

7.5 kg PPG1000 and 15 kg PTMEG2000 and polyoxypropylene triol with a molecular weight of 3,000 were fed into a reactor, heated to 110°C under stirring, dehydrated under vacuum of -0.1 MPa to a moisture content of polyoxypropylene triol of 0.03% or less, and cooled to 40°C. 0.64 kg IPDI and 0.45 kg MDI were fed thereto, heated to 85°C under stirring, maintained at the temperature and reacted for 2 hours, and cooled to 40°C. 4.6 kg IPDI, 1.2 kg MDI and 0.45 kg HMDI were fed thereto, heated to 85°C under stirring, maintained at the temperature and reacted for 2 hours, and cooled to 40°C. 0.66 kg DMPA and 6.5 kg acetone were fed thereto, heated to 80°C under stirring and reacted for 2 hours, and cooled to 50°C. 0.04 kg of a catalyst and 4.3 kg acetone were fed thereto, heated to 75°C under stirring and reacted for 3 hours, and then cooled to 15°C. 0.52 kg triethylamine and 15 kg acetone were added thereto, and stirred for 15 min to obtain a prepolymer.

The prepolymer was transferred to an emulsifier, and the rotation speed of the rotating disk was adjusted to 1300 r/min. 82 kg of an ice-water mixture was added to the prepolymer at a constant speed under high-speed stirring. Stirring was continued for 5 minutes upon opening of the prepolymer, and then the rotation speed was adjusted to 400 r/min. 0.3 kg of a 10X aqueous solution of ethylenediamine was added, and the mixture was stirred for 3 hours and then kept still for 24 hours.

The above emulsion was heated to 40°C, and the acetone in the emulsion was removed under the condition of -0.09 MPa, to prepare waterborne polyurethane emulsion sample 2, with an R value of 1.46.

### Comparative Example 1

The isocyanates in Example 1 were added in one step to obtain Comparative Sample 1.

### Comparative Example 2

The isocyanates in Example 2 were added in one step to obtain Comparative Sample 2.

### Comparative Example 3

The amount of IPDI added for the second time in Example 1 was changed to 5.66 to obtain Comparative Sample 3 with an R value of 1.6.

### Comparative Example 4

The amount of IPDI added for the second time in Example 1 was changed to 4.46 to obtain Comparative Sample 4 with an R value of 1.35.

The present invention will be further described below, with tests of the performance of the waterborne polyurethane condoms prepared from Samples 1 to 3 and Comparative Samples 1 to 4. The test results are shown in Table 1. The polyurethane condom according to the present invention was prepared by the following method: cleaning a mold with pure water, and drying it at 100°C for 5 min; after the mold is cooled to 40°C, dipping it into a waterborne polyurethane emulsion, drying it in an oven at 110°C for 4 min, and repeating this step 3 times; rolling along the mold up to a suitable position after the completion of the above step, cooling the mold after the rolling to 20°C, dipping it into an aqueous solution supplemented with 1% white carbon black, taking it out and drying at a temperature of 140°C for 20 min, and cooling to room temperature and demolding after drying, to obtain a waterborne polyurethane condom.

**Table 1. Table of tested waterborne polyurethane condom performance**

| Sample | Thickness (mm) | Burst pressure (kPa) | Modulus (MPa) | Elongation at break (%) | Tensile strength (MPa) |
|---|---|---|---|---|---|
| Sample 1 | 0.021 | 4.17 | 2.30 | 590 | 50 |
| Sample 2 | 0.021 | 3.82 | 2.50 | 570 | 55 |
| Comparative Sample 1 | 0.021 | 3.66 | 2.10 | 612 | 45 |
| Comparative Sample 2 | 0.021 | 3.22 | 2.33 | 589 | 46 |
| Comparative Sample 3 | 0.021 | 3.98 | 2.75 | 532 | 51 |
| Comparative Sample 4 | 0.021 | 2.94 | 2.02 | 611 | 45.5 |

In the following, the performance test was carried out again after Samples 1 and 2 and Comparative Samples 3 and 4 in the Examples were kept for one week. The test results are shown in Table 2.

**Table 2. Table of waterborne polyurethane condom performance tested after being kept for one week**

| Sample | Thickness (mm) | Burst pressure (kPa) | Modulus (MPa) | Elongation at break (%) | Tensile strength (MPa) |
|---|---|---|---|---|---|
| Sample 1 | 0.021 | 4.13 | 2.2 | 594 | 49.6 |
| Sample 2 | 0.021 | 3.93 | 2.5 | 575 | 55.5 |
| Comparative Sample 3 | 0.021 | 2.96 | 2.2 | 594 | 43 |
| Comparative Sample 4 | 0.021 | 2.21 | 1.72 | 645 | 38 |

It can be seen from the above tables that the moisture absorption and tensile strength of Comparative Sample 3 and Comparative Sample 4 decreased significantly in a later stage, as the storage time increased.

Therefore, the present invention has effectively overcome various deficiency in the prior art and is highly valuable in industrial application. The above examples only illustrate the principles and effects of the present invention, but are not intended to limit the present invention. Anyone skilled in the art can modify or change the above examples without departing from the spirit and scope of the present invention. Therefore, all equivalent modifications or changes made by those skilled in the art without departing from the spirit and technical ideas disclosed in the present invention shall be encompassed by the claims of the present invention.

## Claims

1. A method for preparing a waterborne polyurethane emulsion, **characterized in that** the method comprises at least the steps of:
heating and dehydrating a macromolecular polyol, and then reacting with a first polyisocyanate to obtain a first prepolymer;
reacting the first prepolymer with a second polyisocyanate to obtain a second prepolymer;
reacting the second prepolymer with a hydrophilic chain extender and a small-molecule chain extender to obtain a third prepolymer; and
reacting the third prepolymer with a salt forming agent, followed by emulsification upon addition of a post-chain extender, and by desolvation, to obtain a polyurethane emulsion;
wherein the molar amount of the first polyisocyanate is less than that of the second polyisocyanate, and the molar ratio of the first polyisocyanate to the macromolecular polyol is less than 1:3.

2. The method according to claim 1, **characterized in that** the macromolecular polyol comprises a macromolecular diol and a macromolecular triol, and the mass ratio of the macromolecular diol to the macromolecular triol is (10-15) : (1.5-3.5).

3. The method according to claim 2, **characterized in that** the molecular weight of the macromolecular diol is 1,000 to 3,000, and the molecular weight of the macromolecular triol is 600 to 6,000.

4. The method according to claim 1, **characterized in that** the molar ratio of the macromolecular polyol, the first polyisocyanate and the second polyisocyanate is (1-1.5) : (0.2-0.3) : (2-2.5).

5. The method according to claim 1, **characterized in that** the molar ratio of the hydrophilic chain extender, the small-molecule chain extender and the post-chain extender is (1-1.3) : (0.01-0.05) : (0.5-0.9).

6. The method according to claim 1, **characterized in that** the R value in the waterborne polyurethane emulsion is 1.4-1.6, and the R value is the molar ratio of cyanate groups to hydroxyl groups in the waterborne polyurethane emulsion.

7. A waterborne polyurethane emulsion prepared by the method according to any one of claims 1 to 6.

8. The waterborne polyurethane emulsion according to claim 7, **characterized in that** the waterborne polyurethane emulsion is an anionic waterborne polyurethane emulsion, and the solid content of the waterborne polyurethane emulsion is 30%-35%.

9. A waterborne polyurethane condom prepared from the waterborne polyurethane emulsion according to claim 7 or 8.

10. The polyurethane condom according to claim 9, **characterized in that** the waterborne polyurethane condom has any one or more of the following characteristics:
a thickness of 0.015 to 0.025 mm;
a burst pressure of greater than or equal to 3.0 kPa; and
a tensile strength of greater than or equal to 45 N/mm².
